# EUROPEAN PATENT SPECIFICATION

(11) **EP 3 887 099 B1**
(45) Date of publication and mention of the grant of the patent: **12.03.2025**
(21) Application number: 19831882.6
(22) Date of filing: 26.11.2019
(51) Int. Cl.: B25J 9/00, B25J 9/10

(54) **WEARABLE ROBOTIC DEVICE FOR MOVING A USER**
AM KÖRPER TRAGBARE ROBOTERVORRICHTUNG ZUM BEWEGEN EINES BENUTZERS
DISPOSITIF ROBOTIQUE PORTABLE DESTINÉ AU DÉPLACEMENT D'UN UTILISATEUR

(30) Priority: 27.11.2018 IT 201800010633
(43) Date of publication of application: 06.10.2021
(73) Proprietor: Scuola Superiore di Studi Universitari e di Perfezionamento Sant'Anna, 56127 Pisa (IT)
(72) Inventor: BALDONI, Andrea, 56025 Pontedera (PI) (IT); FANTOZZI, Matteo, 55049 Viareggio (LU) (IT); CREA, Simona, 55100 Lucca (IT); VITIELLO, Nicola, 56025 Pontedera (PI) (IT)
(74) Representative: ABM Agenzia Brevetti & Marchi
(86) International application number: PCT/IB2019/060177
(87) International publication number: WO 2020/109996

(56) References cited:
- EP-A2- 2 942 162
- US-A1- 2011 040 216
- US-A1- 2013 046 218
- US-A1- 2014 277 739
- US-A1- 2016 158 032

## Description

### Field of the invention

The present invention relates to the field of robotic devices for assisting the movement of a user.

In particular, the invention relates to a robotic device for the improvement of a prosthesis or an exoskeleton of a lower limb.

### Description of the prior art

As known, different foot, ankle or knee prostheses may be prescribed to provide adequate limb amputees.

For a basic functionality, that is to get up and be able to walk, a SACH (Solid Ankle Cushioned Heel) foot is adopted. It is a stiff foot that offers only support and no energy contribution to walking. The amputee will have to compensate with the residual limbs for the absence of energy supply, at the expense of speed and metabolic consumption.

An increase in mobility can be obtained by using an ESAR (Energy Storage And Return) foot, comprising an elastic carbon structure capable of storing mechanical energy during the first part of the support phase (*weight acceptance*) of the walk and releasing it in the push phase (*push-off*).

However, a user's step requires between 0.2 and 0.29 J/Kg of mechanical energy and the aforementioned prostheses are able to generate between 0.06 and 0.11 J/Kg. The amount of energy missing must therefore be compensated by the other joint joints.

A possible alternative lies in the use of a mechanical actuator that inserts active power into the step cycle, transforming the passive prosthesis into an active one, and allowing the user not to have to compensate for the missing energy with muscle energy.

However, active prostheses have several disadvantages, since mechanical and electronic components are generally very complex and heavy. This entails higher costs and less reliability than passive prostheses. In addition, in the event of a malfunction or a lack of electricity, these prostheses lose all their functionality becoming rigid and heavy appendages that can also totally block the user's mobility.

US2013046218 describes a device for the accumulation and release of mechanical energy capable of assisting a user's walk. The device has a frame comprising an upper portion adapted to bind to the user's calf and a lower portion adapted to bind to the foot in a rotatable manner. A rotating clutch and an elastic element are also provided which allow the controlled release of mechanical energy to move the two portions of the frame.

US 2016/158032 A1 forms also part of the prior art disclosing the preamble of claim 1.

However, in this device the release of mechanical energy takes place simply at the end of the accumulation phase and is in no way related to the patient's step phase. This means that the impulse provided by the elastic element is not synchronized with the user's pace, significantly reducing the quality of motor assistance provided by the device.

### Summary of the invention

It is therefore a feature of the present invention to provide a robotic device for the movement of a user that has the practicality of using a prosthesis or a passive exoskeleton, who however does not need energy compensation by the user to obtain sufficient thrust to perform a step.

It is also a feature of the present invention to provide such a robotic device that can be used both on a foot prosthesis and on an exoskeleton for assisting a user's foot.

It is also a feature of the present invention to provide such a robotic device that can adapt to a variety of types of gait and of balancing the weight of a user.

These and other objects are achieved by a robotic device for the movement of a user according to claim 1.

Other aspects of the invention are described in the claims from 2 to 13.

### Brief description of the drawings

Further characteristic and/or advantages of the present invention are more bright with the following description of an exemplary embodiment thereof, exemplifying but not limitative, with reference to the attached drawings in which:
- Fig. 1A shows a kinematic diagram of the robotic device according to the present invention;
- Fig. 1B shows the kinematic diagram of Fig. 1A when the relative movement ***s*** is equal to the predetermined threshold value ***s**** and the robotic device passes from the first to the second configuration;
- Fig. 2A shows a kinematic diagram of the robotic device wherein the interface frame and the lower portion are connected by a laminar resilient element;
- Fig. 2B shows a kinematic diagram of the robotic device wherein the interface frame and the lower portion are connected by a rotational joint;
- Fig. 3 diagrammatically shows a first exemplary embodiment of the robotic device wherein the lower portion and the resilient element make up a foot prosthesis;
- Fig. 4A shows the embodiment of Fig. 3 in the first configuration during the deformation of the elastic element, until it reaches the threshold value;
- Fig. 4B shows the embodiment of Fig. 3 in the moment in which, passing from the first to the second configuration, there is an instantaneous releasing of elastic energy;
- Fig. 5 shows a possible real application of the embodiment of Fig. 3;
- Fig. 6A shows an embodiment of the robotic device having a linear spring and a rotational switching device;
- Fig. 6B shows an embodiment of the robotic device having a linear spring and a linear switching device;
- Fig. 7A shows an embodiment of the robotic device having a torsional spring and a linear switching device;
- Fig. 7B shows an embodiment of the robotic device having a torsional spring and a rotational switching device;
- Fig. 8 shows an embodiment of the robotic device similar to that of Fig. 6A wherein the lower portion is a belt of engagement to a foot of the user.

### Description of a preferred exemplary embodiment

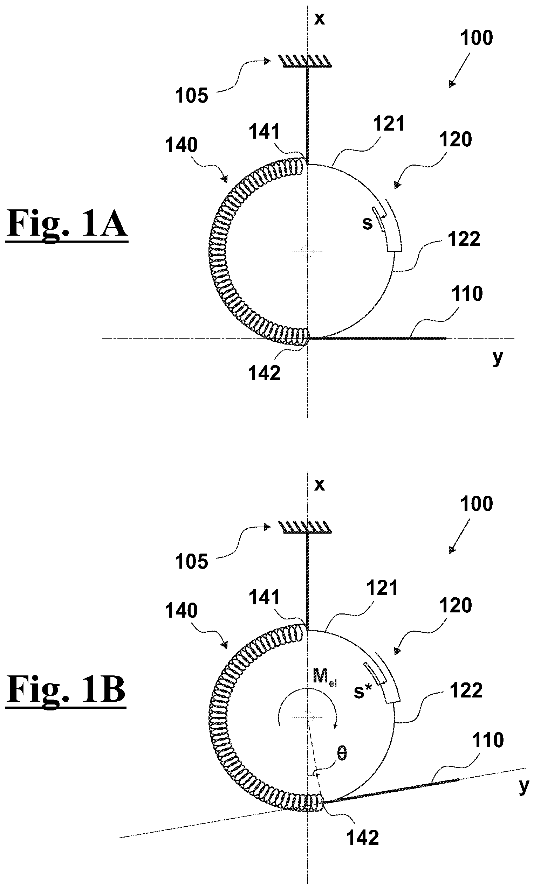

With reference to Figs. 1A and 1B, the robotic device 100, according to the present invention, comprises an interface frame 105, arranged to connect the robotic device 100 to a lower limb or to a lower limb prosthesis, a lower portion 110 and a resilient element 140 having a first end 141 connected to the interface frame 105 and a second end 142 connected to the lower portion 110.

Considering an axis ***x*** integral to the interface frame 105 and an axis ***y*** integral to the lower portion 110, the resilient element 140 can be deformed as a function of an angular variation ***θ*** between the axis ***x*** and the axis ***y,*** as shown in Fig. 1B.

The robotic device 100 also comprises a switching device 120 comprising a first element 121, connected to the interface frame 105, and a second element 122, connected to the lower portion 110. In particular, the first and the second element 121,122 are adapted to carry out a relative movement ***s*** as a function of the angular variation ***θ*** between the axis ***x*** and the axis ***y*.**

This way, when the angular variation ***θ*** increases, the resilient element 140 deforms storing elastic energy and, at the same time, increases also the relative movement ***s*** between the elements 121 and 122. In this step the robotic device 100 is in a first configuration, or energy storage configuration.

With reference to Fig. 1,B, when the relative movement ***s*** is equal to a predetermined threshold value ***s**,** the robotic device 100 is configured to pass from the first configuration to a second configuration, where the resilient element 140 instantly releases elastic energy creating an angular moment ***Mₑₗ*** arranged to oppose the increasing of the angular variation ***θ*.**

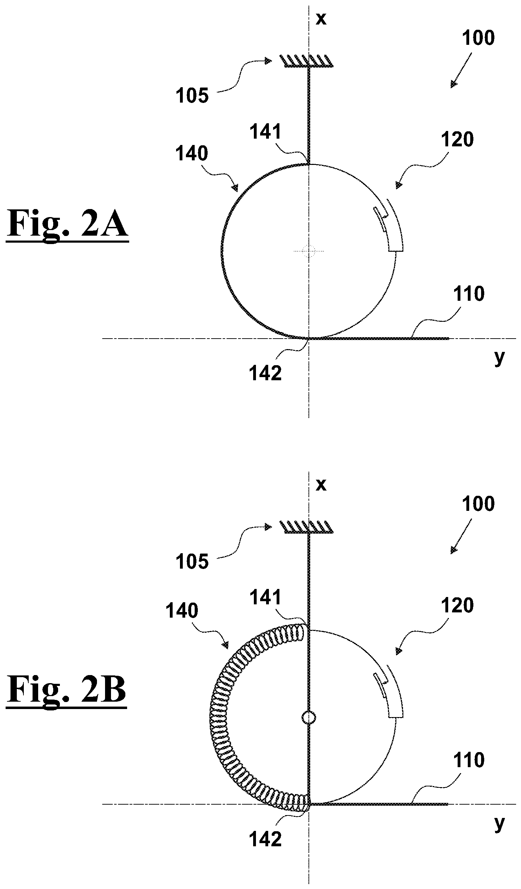

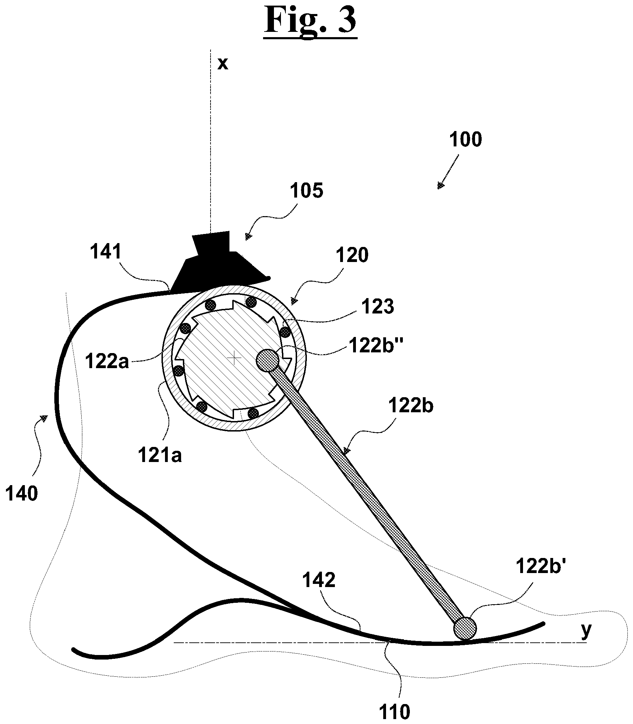

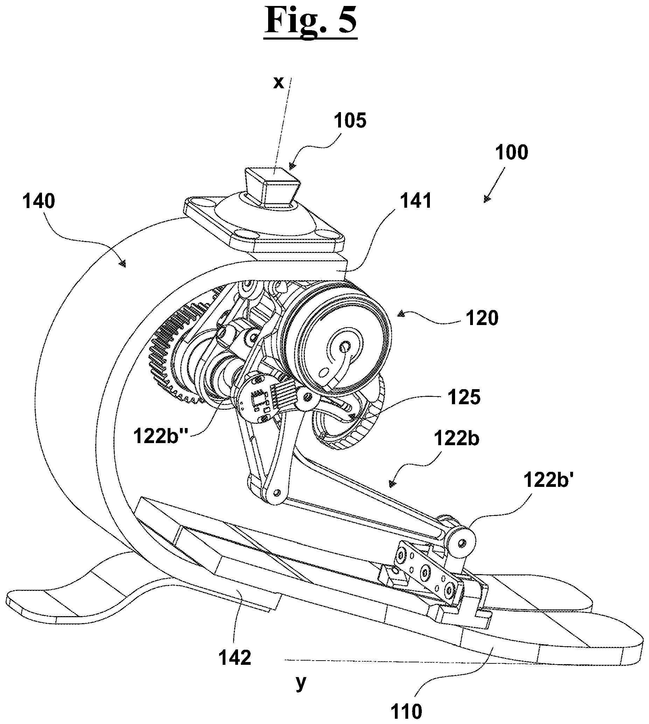

In Fig. 2A a kinematic diagram of the robotic device 100 is shown wherein the interface frame 105 and the lower portion 110 are connected by a laminar resilient element 140. Such kinematic diagram is at the basis of the operation of the foot prosthesis diagrammatically shown in Fig. 3 and, in a way more detailed, for example, in the embodiment of Fig. 5, wherein the lower portion 110 and the resilient element 140 make up a passive foot prosthesis, present in prior art.

In particular, in Figs. 3 and 5, the first element 121 of the switching device 120 comprises a cylindrical housing 121a, whereas the second element 122 comprises a free wheel 122a and a rigid arm 122b comprising a first end 122b' connected to the lower portion 110 and a second end 122b'' connected to the free wheel 122a.

The switching device 120 also comprises a plurality of balls 123 that, with the free wheel 122a and the cylindrical housing 121a, provides a unidirectional clutch where the free wheel 122a can carry out a rotational relative movement ***s*** with respect to the cylindrical housing 121a only in one direction of rotation, in particular in Fig. 3 counter clockwise with respect to the plane of the sheet.

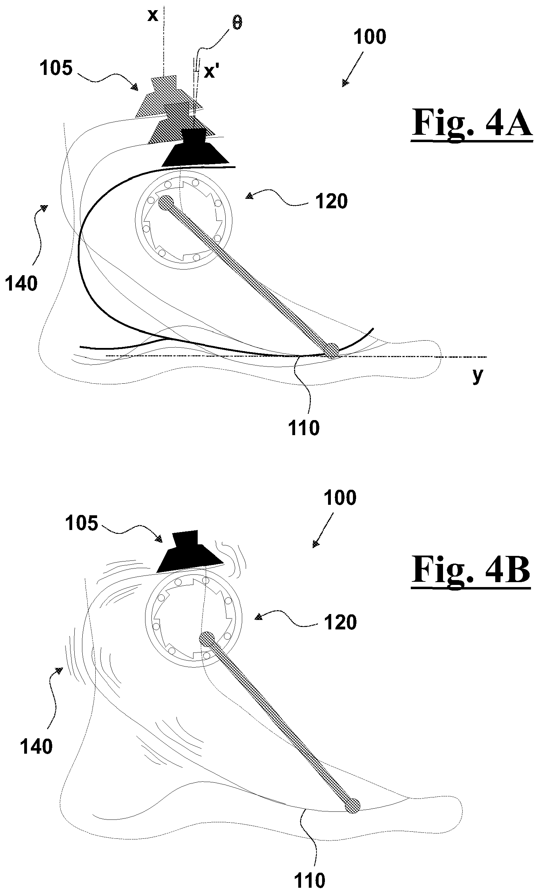

In the figures 4A and 4B are shown, respectively, the first configuration, of storage of elastic energy, and the second configuration, of instantaneous release of this energy. For the sake of clearness of the drawing, the axis ***y*** is kept with a constant direction during the deformation of the resilient element 140, whereas the axis ***x*** changes its own direction, becoming the axis ***x'*,** allowing to highlight the angular variation ***θ*.**

In particular, in Fig. 4A shows the successive deformations of the resilient element 140 due to the movement of the user's weight on the lower portion 110. During the deformation, and the increasing the angular variation ***θ*,** the rigid arm 122b pushes towards the frame 105 its second end 122b'' bringing the free wheel 122a to carry out a relative rotation ***s*** in the only direction in which the rotation is allowed by the unidirectional clutch.

If the deformation stopped before the movement ***s*** reached the threshold value ***s**,** the switching device 120, owing to the unidirectional clutch, would prevent the resilient element 140 from resuming its shape at rest. Therefore, as long as the robotic device 100 is in the first configuration, i.e. before the movement ***s*** reaches the threshold ***s**,** the resilient element 140 accumulates elastic energy irreversibly.

when the angular variation ***θ*** is such as to allow the second end 122b'' to make a relative movement ***s* ≥ *s**,** i.e. when the rigid arm 122b overlaps the centre of the free wheel 122a, the robotic device 100 passes into the second configuration, as shown in Fig. 4B, instantly releasing the accumulated elastic energy.

This elastic energy, released impulsively, allows to provide the user with a sufficient push to take the step without having to compensate for the missing energy with muscle energy or through an external actuator. In fact, with respect to a passive resilient foot prosthesis of the prior art, the embodiment of the device 100 of Figs. 3-5, owing to the presence of the switching device 120, is adapted to control the release of the elastic energy and to supply it to the user exactly in the instant wherein the pushing step of the foot begins, effectively replacing the impulse generally provided by the muscles of the leg.

With reference even to Fig. 5, the device 100 can also comprise adjustment means, such as the elongated hole 125, capable of adjusting the threshold value ***s**,** at which an impulsive release of elastic energy is obtained. This makes it possible to adapt the robotic device 100 to different types of walking and balancing the weight of a user.

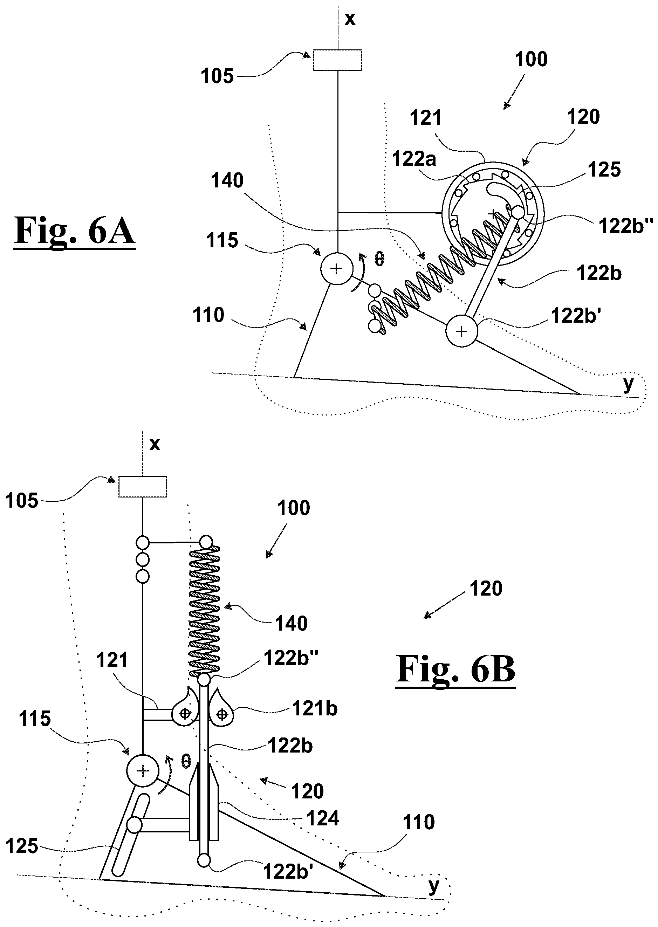

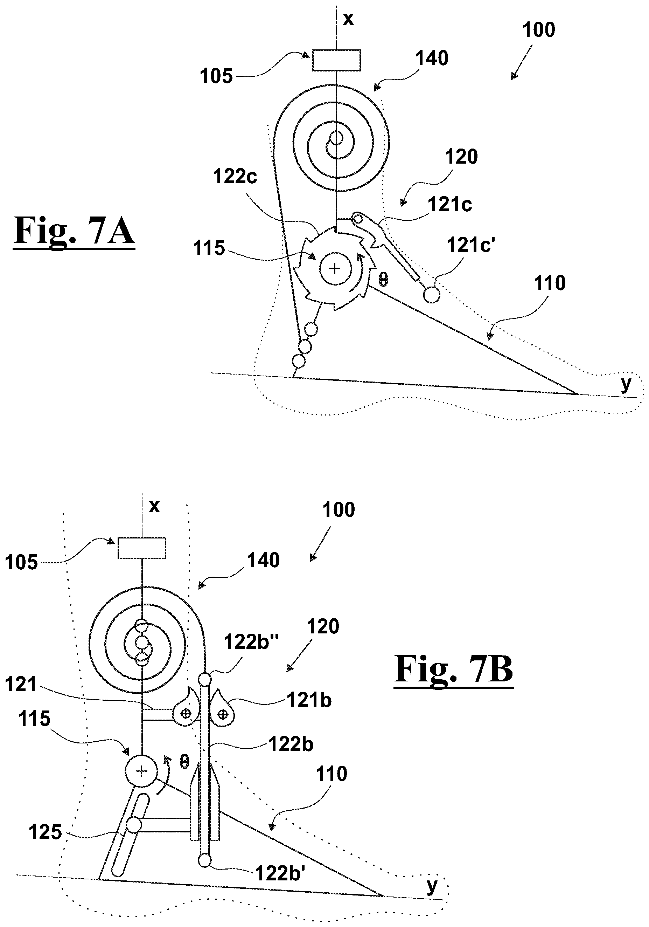

With reference to Figs. 6A, 6B, 7A and 7B, some variants of the robotic device 100 are shown, in which different embodiments of the resilient element 140 and of the switching device 120 and different combinations of these embodiments are provided.

All the embodiments of Figs. 6A, 6B, 7A and 7B may have application as an exoskeleton or as a foot prosthesis, depending on whether the lower portion 110 is, respectively, a means of engagement with the foot of the user or a prosthetic foot.

In particular, in Fig. 6A an embodiment is shown similar to that of Figs. 3-5, wherein a linear spring 140 and a "clutch" type switching device 120, i.e. comprising a rotational unidirectional clutch, are provided.

In Fig. 6B instead an embodiment is shown wherein the switching device 120 is of the type linear and comprises a rigid arm 122b, a wedge element 124 and two rotating elements 121b having a profile such as to allows a clamping by friction of the rotating elements 121b on said rigid arm 122b in a single direction of relative movement. For example, the rotating elements 121b may have logarithmic spiral shape. When there is an angular variation ***θ*** of the axis ***y*,** and therefore of the portion 110, arm 122b moves upwards compressing the spring 140. In particular, the rigid arm 122b carries out a translation ***s*** with respect to the rotating elements 121b that, owing to the particular spiral-shaped profile, allow this translation upwards but prevent it in an opposite direction owing to the clamping friction.

As in the previous embodiment, therefore, as long as the robotic device 100 is in the first configuration, i.e. before the movement ***s*** reaches the threshold ***s****, the resilient element 140 accumulates elastic energy irreversibly. When the translation ***s*** is such that the wedge element 124 comes to touch the rotating elements 121b, there is ***s* = *s**** and the rotating elements 121b loose contact with the rigid arm 122b, instantly allowing the translation downwards and therefore the release of elastic energy necessary for the user's movement.

The elongated hole 125 also allows you to adjust the relative position between the rigid arm 122b and the wedge element 124, allowing you to change the threshold value ***s**** and to adapt it to the specific needs of the user.

In Fig. 7A there is another embodiment where the resilient element 140 is a torsional spring, whereas the switching device 120 comprises a pawl 121c and a gear 122c arranged to form a ratchet mechanism. Even in this case, when there is an angular variation ***θ*,** the spring 140 accumulates elastic energy and the toothed wheel 122c can rotate only in one direction due to the pawl 121c that is engages on the teeth of the wheel itself. When then the angular variation ***θ*** is such that the portion 110 contacts the ball 121c', the threshold ***s**** is reached and the pawl 121c rises, allowing the instantaneous release of the elastic energy.

In Fig. 7B there is a further embodiment which combines the torsional spring 140 of Fig. 7A with the linear switching device 120 of Fig. 6B.

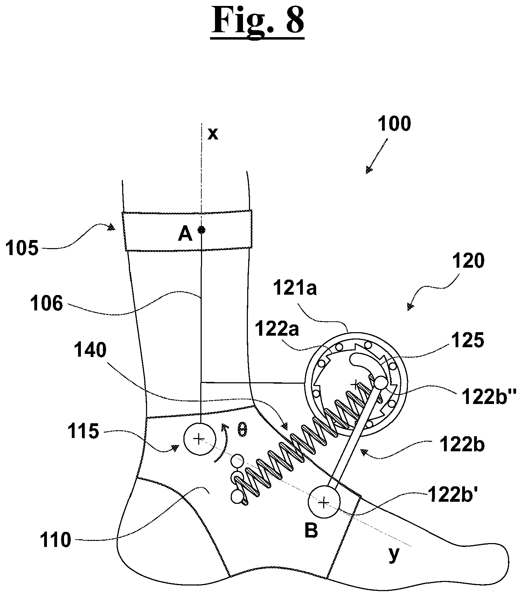

In Fig. 8 a possible embodiment is shown of the present invention, similar to that of Fig. 6A, wherein the lower portion 120 is a belt of engagement to a foot of the user. Alternatively, the lower portion 120 can be a shoe, or any other element that allows to make the axis *y* integral with the foot of the user.

## Claims

1. A robotic device (100) for the movement of a user, said robotic device (100) comprising:
- an interface frame (105) arranged to connect said robotic device (100) to a lower limb or to a lower limb prosthesis, said interface frame (105) being integral to an axis ***x*;**
- a lower portion (110) integral to an axis ***y*;**
- a resilient element (140) having a first end (141) connected to said interface frame (105) and a second end (142) connected to said lower portion (110), said resilient element (140) being arranged to deform as a function of an angular variation ***θ*** between said axis ***x*** and said axis ***y*;**
- a switching device (120) comprising a first element (121), connected to said interface frame (105), and a second element (122), connected to said lower portion (110), said first and second element (121,122) arranged to carry out a relative movement ***s*** as a function of said angular variation ***θ*** between said axis ***x*** and said axis ***y*;** said robotic device (100) being configured in such a way that:
- when said relative movement s is less than a predetermined threshold value ***s**** and said angular variation ***θ*** increases, said robotic device (100) is in a first configuration and said resilient element (140) deforms storing elastic energy, said switching device (120) being configured in such a way that in said first configuration said relative movement ***s*** can take place only in one direction of motion; said robotic device (100) being **characterized in that** it is configured in such a way that:
- when said relative movement ***s*** is equal to said predetermined threshold value ***s**,** as consequence of the condition ***s* = *s**** said robotic device (100) passes in a second configuration and said resilient element (140) instantly releases elastic energy creating an angular moment ***Mₑₗ*** arranged to oppose the increasing of said angular variation ***θ*.**

2. The robotic device (100), according to claim 1, wherein said interface frame (105) is constrained to the shin of said user and said lower portion (110) is constrained to the foot of said user.

3. The robotic device (100), according to claim 1, wherein said lower portion (110) is a foot prosthesis arranged to support a variable fraction of weight ***Pᵥ*** of said user.

4. The robotic device (100), according to claim 1, wherein said resilient element (140) is a linear spring.

5. The robotic device (100), according to claim 1, wherein said resilient element (140) is a torsional spring.

6. The robotic device (100), according to claim 1, wherein said first element (121) comprises a cylindrical housing (121a) and said second element (122) comprises a free wheel (122a) arranged to carry out a rotational relative movement ***s*** with respect to said cylindrical housing (121a).

7. The robotic device (100), according to claim 1, wherein said second element (122) comprises a rigid arm (122b) comprising a first end (122b') connected to said lower portion (110) and a second end (122b'') for carrying out said relative movement ***s*** as a function of said angular variation ***θ*.**

8. The robotic device (100), according to claims 6 and 7, wherein said second end (122b") is connected to said free wheel (122a).

9. The robotic device (100), according to claim 1, wherein said first element (121) comprises a pawl (121c) and said second element (122) comprises a gear (122c) arranged to carry out a rotational relative movement ***s*** with respect to said first element (121), said gear (122c) and said pawl (121c) arranged to provide a ratchet mechanism.

10. The robotic device (100), according to claim 7, wherein said first element (121) comprises at least two rotating elements (121b) and said second element (122) comprises a rigid arm (122b), said rigid arm (122b) for carrying out a rectilinear relative movement ***s*** with respect to said rotating elements (121b), said rotating elements (121b) being configured to allow said rectilinear relative movement ***s*** in a first direction of motion and arranged to clamp by friction on said rigid arm (122b) for preventing said rectilinear relative movement s in a second direction of motion opposite to said first direction of motion.

11. The robotic device (100), according to claim 1, wherein said switching device (120) comprises an adjustment means (125) arranged to adjust said threshold value ***s**.**

12. The robotic device (100), according to claim 11, wherein said adjustment means (125) comprises at least one elongated hole.

13. The robotic device (100), according to claim 11, wherein at least one sensor and an actuator are comprised, said actuator arranged to adjust said relative movement ***s*** for causing said robotic device (100) to pass from said first configuration to said second configuration in consequence of a signal emitted by said at least one sensor.

## Patentansprüche

1. Robotervorrichtung (100) zur Bewegung eines Benutzers, wobei die Robotervorrichtung (100) Folgendes umfasst:
- einen Schnittstellenrahmen (105), der dazu ausgelegt ist, die Robotervorrichtung (100) mit einer unteren Extremität oder einer Prothese der unteren Extremität zu verbinden, wobei der Schnittstellenrahmen (105) ein integraler Bestandteil einer Achse x ist;
- einen unteren Abschnitt (110), der ein integraler Bestandteil einer Achse *y* ist;
- ein elastisches Element (140) mit einem ersten Ende (141), das mit dem Schnittstellenrahmen (105) verbunden ist, und einem zweiten Ende (142), das mit dem unteren Abschnitt (110) verbunden ist, wobei das elastische Element (140) dazu ausgelegt ist, sich in Abhängigkeit von einer Winkelabweichung ***θ*** zwischen der Achse x und der Achse *y* zu verformen;
- eine Schaltvorrichtung (120), die ein erstes Element (121), das mit dem Schnittstellenrahmen (105) verbunden ist, und ein zweites Element (122) umfasst, das mit dem unteren Abschnitt (110) verbunden ist, wobei das erste und das zweite Element (121, 122) dazu ausgelegt sind, eine Relativbewegung ***s*** in Abhängigkeit der Winkelabweichung ***θ*** zwischen der Achse ***x*** und der Achse ***y*** auszuführen;
wobei die Robotervorrichtung (100) derart konfiguriert ist, dass:
- wenn die Relativbewegung ***s*** kleiner als ein vorbestimmter Schwellenwert ***s**** ist und die Winkelabweichung ***θ*** zunimmt, sich die Robotervorrichtung (100) in einer ersten Konfiguration befindet und sich das elastische Element (140) verformt und elastische Energie speichert, wobei die Schaltvorrichtung (120) derart konfiguriert ist, dass in der ersten Konfiguration die Relativbewegung s nur in einer Bewegungsrichtung erfolgen kann; wobei die Robotervorrichtung (100) **dadurch gekennzeichnet ist, dass** sie derart konfiguriert ist, dass:
- wenn die Relativbewegung **s** gleich dem vorbestimmten Schwellenwert ***s**** ist, die Robotervorrichtung (100) als Folge der Bedingung ***s = s**** in eine zweite Konfiguration übergeht und das elastische Element (140) augenblicklich elastische Energie freisetzt, wodurch ein Winkelmoment ***Mₑₗ*** erzeugt wird, das dazu ausgelegt ist, der Vergrößerung der Winkelabweichung ***θ*** entgegenzuwirken.

2. Robotervorrichtung (100) nach Anspruch 1, wobei der Schnittstellenrahmen (105) an dem Schienbein des Benutzers befestigt ist und der untere Abschnitt (110) an dem Fuß des Benutzers befestigt ist.

3. Robotervorrichtung (100) nach Anspruch 1, wobei der untere Abschnitt (110) eine Fußprothese ist, die dazu ausgelegt ist, einen variablen Anteil des Gewichts ***Pᵥ*** des Benutzers zu stützen.

4. Robotervorrichtung (100) nach Anspruch 1, wobei das elastische Element (140) eine lineare Feder ist.

5. Robotervorrichtung (100) nach Anspruch 1, wobei das elastische Element (140) eine Torsionsfeder ist.

6. Robotervorrichtung (100) nach Anspruch 1, wobei das erste Element (121) ein zylindrisches Gehäuse (121a) umfasst und das zweite Element (122) ein Freilaufrad (122a) umfasst, das dazu ausgelegt ist, eine Relativdrehbewegung ***s*** in Bezug auf das zylindrische Gehäuse (121a) auszuführen.

7. Robotervorrichtung (100) nach Anspruch 1, wobei das zweite Element (122) einen starren Arm (122b) umfasst, der ein erstes Ende (122b'), das mit dem unteren Abschnitt (110) verbunden ist, und ein zweites Ende (122b") zum Ausführen der Relativbewegung ***s*** in Abhängigkeit der Winkelabweichung ***θ*** umfasst.

8. Robotervorrichtung (100) nach den Ansprüchen 6 und 7, wobei das zweite Ende (122b") mit dem Freilaufrad (122a) verbunden ist.

9. Robotervorrichtung (100) nach Anspruch 1, wobei das erste Element (121) eine Sperrklinke (121c) umfasst und das zweite Element (122) ein Sperrrad (122c) umfasst, das dazu ausgelegt ist, eine Relativdrehbewegung ***s*** in Bezug auf das erste Element (121) auszuführen, wobei das Sperrrad (122c) und die Sperrklinke (121c) dazu ausgelegt sind, einen Ratschenmechanismus bereitzustellen.

10. Robotervorrichtung (100) nach Anspruch 7, wobei das erste Element (121) mindestens zwei rotierende Elemente (121b) umfasst und das zweite Element (122) einen starren Arm (122b) umfasst, wobei der starre Arm (122b) zum Ausführen einer geradlinigen Relativbewegung s in Bezug auf die rotierenden Elemente (121b) dient, wobei die rotierenden Elemente (121b) dazu konfiguriert sind, die geradlinige Relativbewegung ***s*** in einer ersten Bewegungsrichtung zu ermöglichen, und dazu ausgelegt sind, sich durch Reibung an dem starren Arm (122b) festzuklemmen, um die geradlinige Relativbewegung ***s*** in einer zweiten Bewegungsrichtung, die der ersten Bewegungsrichtung entgegengesetzt ist, zu verhindern.

11. Robotervorrichtung (100) nach Anspruch 1, wobei die Schaltvorrichtung (120) ein Einstellmittel (125) umfasst, das zum Einstellen des Schwellenwerts ***s**** ausgelegt ist.

12. Robotervorrichtung (100) nach Anspruch 11, wobei das Einstellmittel (125) mindestens ein Langloch umfasst.

13. Robotervorrichtung (100) nach Anspruch 11, wobei mindestens ein Sensor und ein Aktuator umfasst sind, wobei der Aktuator dazu ausgelegt ist, die Relativbewegung s so einzustellen, dass bewirkt wird, dass die Robotervorrichtung (100) infolge eines durch den mindestens einen Sensor ausgesendeten Signals von der ersten Konfiguration in die zweite Konfiguration übergeht.

## Revendications

1. Dispositif robotique (100) destiné au mouvement d'un utilisateur, ledit dispositif robotique (100) comprenant :
- un cadre d'interface (105) agencé pour relier ledit dispositif robotique (100) à un membre inférieur ou à une prothèse de membre inférieur, ledit cadre d'interface (105) étant solidaire d'un axe ***x* ;**
- une partie inférieure (110) solidaire d'un axe ***y** ;*
- un élément élastique (140) comportant une première extrémité (141) reliée audit cadre d'interface (105) et une seconde extrémité (142) reliée à ladite partie inférieure (110), ledit élément élastique (140) étant agencé pour se déformer en fonction d'une variation angulaire ***θ*** entre ledit axe ***x*** et ledit axe ***y*** ;
- un dispositif de commutation (120) comprenant un premier élément (121), relié audit cadre d'interface (105), et un second élément (122), relié à ladite partie inférieure (110), lesdits premier et second éléments (121, 122) étant agencés pour effectuer un mouvement relatif ***s*** en fonction de ladite variation angulaire ***θ*** entre ledit axe ***x*** et ledit axe ***y*** ;
ledit dispositif robotique (100) étant configuré d'une telle façon que :
- lorsque ledit mouvement relatif s est inférieur à une valeur seuil prédéfinie ***s**** et ladite variation angulaire ***θ*** augmente, ledit dispositif robotique (100) est dans une première configuration et ledit élément élastique (140) se déforme en stockant de l'énergie élastique, ledit dispositif de commutation (120) étant configuré d'une telle façon que, dans ladite première configuration, ledit mouvement relatif s ne peut avoir lieu que dans une seule direction de mouvement ; ledit dispositif robotique (100) étant **caractérisé en ce qu'**il est configuré d'une telle façon que :
- lorsque ledit mouvement relatif ***s*** est égal à ladite valeur seuil prédéfinie ***s**,** en conséquence de la condition ***s* = *s**** ledit dispositif robotique (100) passe dans une seconde configuration et ledit élément élastique (140) libère instantanément une énergie élastique créant un moment angulaire ***Mₑₗ*** agencé pour s'opposer à l'augmentation de ladite variation angulaire ***θ*.**

2. Dispositif robotique (100) selon la revendication 1, ledit cadre d'interface (105) étant contraint au tibia dudit utilisateur et ladite partie inférieure (110) étant contrainte au pied dudit utilisateur.

3. Dispositif robotique (100) selon la revendication 1, ladite partie inférieure (110) étant une prothèse de pied agencée pour supporter une fraction variable du poids ***Pᵥ*** dudit utilisateur.

4. Dispositif robotique (100) selon la revendication 1, ledit élément élastique (140) étant un ressort linéaire.

5. Dispositif robotique (100) selon la revendication 1, ledit élément élastique (140) étant un ressort de torsion.

6. Dispositif robotique (100) selon la revendication 1, ledit premier élément (121) comprenant un logement cylindrique (121a) et ledit second élément (122) comprenant une roue libre (122a) agencée pour effectuer un mouvement relatif de rotation s par rapport audit logement cylindrique (121a).

7. Dispositif robotique (100) selon la revendication 1, ledit second élément (122) comprenant un bras rigide (122b) comprenant une première extrémité (122b') reliée à ladite partie inférieure (110) et une seconde extrémité (122b") pour effectuer ledit mouvement relatif s en fonction de ladite variation angulaire ***θ*.**

8. Dispositif robotique (100) selon les revendications 6 et 7, ladite seconde extrémité (122b") étant reliée à ladite roue libre (122a).

9. Dispositif robotique (100) selon la revendication 1, ledit premier élément (121) comprenant un cliquet (121c) et ledit second élément (122) comprenant une roue dentée (122c) agencée pour effectuer un mouvement relatif de rotation ***s*** par rapport audit premier élément (121), ladite roue dentée (122c) et ledit cliquet (121c) étant agencés pour fournir un mécanisme à cliquet.

10. Dispositif robotique (100) selon la revendication 7, ledit premier élément (121) comprenant au moins deux éléments rotatifs (121b) et ledit second élément (122) comprenant un bras rigide (122b), ledit bras rigide (122b) étant destiné à effectuer un mouvement relatif rectiligne ***s*** par rapport auxdits éléments rotatifs (121b), lesdits éléments rotatifs (121b) étant configurés pour permettre ledit mouvement relatif rectiligne ***s*** dans une première direction de mouvement et agencés pour serrer par frottement sur ledit bras rigide (122b) pour empêcher ledit mouvement relatif rectiligne s dans une seconde direction de mouvement opposée à ladite première direction de mouvement.

11. Dispositif robotique (100) selon la revendication 1, ledit dispositif de commutation (120) comprenant un moyen de réglage (125) agencé pour régler ladite valeur seuil ***s*.***

12. Dispositif robotique (100) selon la revendication 11, ledit moyen de réglage (125) comprenant au moins un trou allongé.

13. Dispositif robotique (100) selon la revendication 11, au moins un capteur et un actionneur étant compris, ledit actionneur étant agencé pour régler ledit mouvement relatif s afin d'amener ledit dispositif robotique (100) à passer de ladite première configuration à ladite seconde configuration en conséquence d'un signal émis par ledit au moins un capteur.
